# EUROPEAN PATENT SPECIFICATION

(11) **EP 3 510 937 B1**
(45) Date of publication and mention of the grant of the patent: **05.10.2022**
(21) Application number: 17848370.7
(22) Date of filing: 05.06.2017
(51) Int. Cl.: A61B 8/08, A61B 8/14

(54) **ULTRASONIC DIAGNOSIS DEVICE AND OPERATION METHOD THEREFOR**
ULTRASCHALLDIAGNOSEVORRICHTUNG UND BETRIEBSVERFAHREN DAFÜR
DISPOSITIF ULTRASONORE ET PROCÉDÉ DE FONCTIONNEMENT ASSOCIÉ

(30) Priority: 09.09.2016 JP 2016176590
(43) Date of publication of application: 17.07.2019
(73) Proprietor: FUJIFILM Healthcare Corporation, Kashiwa-shi, Chiba 277-0804 (JP)
(72) Inventor: TOYOMURA, Takashi, Tokyo 100-8280 (JP); NOGUCHI, Yoshimi, Tokyo 100-8280 (JP); TAKANOHASHI, Kenta, Tokyo 100-8280 (JP); INOUE, Nobuyasu, Tokyo 100-8280 (JP)
(74) Representative: Mewburn Ellis LLP
(86) International application number: PCT/JP2017/020825
(87) International publication number: WO 2018/047425

(56) References cited:
- CN-A- 105 662 474
- JP-A- 2015 171 476
- JP-A- 2016 067 814
- JP-A- 2016 140 588
- YU ET AL: "Fetal Abdominal Contour Extraction and Measurement in Ultrasound Images", ULTRASOUND IN MEDICINE AND BIOLOGY, NEW YORK, NY, US, vol. 34, no. 2, 23 October 2007 (2007-10-23), pages 169-182, XP022434024, ISSN: 0301-5629, DOI: 10.1016/J.ULTRASMEDBIO.2007.06.026
- TOMONARI MASUZAKI et al.: "High Accuracy Ellipse- Specific Fitting", IPSJ SIG Technical Report, 2012, pages 1-11, XP055579273,

## Description

### Technical Field

The present invention relates to an ultrasonic diagnosis device and an operation method for the device, and more particularly, to an image processing technique for tomographic images used in diagnosis.

### Background Art

In a fetus diagnosis using an ultrasonic diagnosis device, to check whether or not a fetus normally grows, in some cases, the abdomen circumference is measured by using a tomographic image. Patent Literature 1 discloses, as a method for automatically measuring an abdomen circumference in a tomographic image, a method of extracting an abdomen contour region with Adaboost constructing a strong discriminator as a combination of plural weak discriminators.

### Citation List

### Patent Literature

PTL 1: CN103239249

### Summary of Invention

### Technical Problem

In Patent Literature 1, the abdomen contour region is detected with the Adaboost, and an ellipse is fitted to edge feature points extracted in the region, and the circumference is measured. However, in some cases, a tomographic image obtained on an actual clinical site has an unclear contour. When it is impossible to sufficiently extract edge feature points in the detected abdomen contour region, it is difficult to obtain an accurate circumference.

The non-patent document by Yu et al.: "Fetal Abdominal Contour Extraction and Measurement in Ultrasound Images", ULTRASOUND IN MEDICINE AND BIOLOGY, vol. 34, no. 2, pages 169-182 discloses fetal abdominal contour extraction and measurement using several image segmentation techniques.

Accordingly, the object of the present invention is to solve the above-described problem, and to provide an ultrasonic diagnosis device capable of more accurately measuring an abdomen circumference, and an operation method for the device. Solution to Problem

To attain the above-described object, the present invention provides an ultrasonic diagnosis device comprising: an ultrasonic probe part that transmits/receives an ultrasonic wave; an image processing unit that generates a tomographic image of a tissue in a test subject based on a signal obtained from the ultrasonic probe part; an abdomen circumference measurement unit that generates a plurality of ellipses corresponding to an abdomen of the test subject from the tomographic image, and measures a circumference of the abdomen; and an output unit that displays a measurement result from the abdomen circumference measurement unit.

Further, to attain the above-described object, the present invention provides an operation method for an ultrasonic diagnosis device having a processor, an output unit and an input unit, wherein the processor generates a plurality of ellipses corresponding an abdomen of a test subject, from a tomographic image of a tissue in the test subject, generated based on a signal obtained from an ultrasonic probe part that transmits/receives an ultrasonic wave, measures the circumference of the abdomen a plural number of times, and outputs a plurality of measurement results to the output unit.

### Advantageous Effects of Invention

According to the present invention, it is possible for a user to select a more appropriate abdomen circumference by measuring circumferences of plural ellipses as candidates with respect to an abdomen contour.

### Brief Description of Drawings

Figure 1 is a block diagram showing an example of a configuration of an ultrasonic diagnosis device according to an example 1.
Figure 2 is a block diagram showing an example of a configuration of an abdomen circumference measurement unit according to the example 1.
Figure 3 is a flowchart showing an example of a procedure performed with an ellipse fitting unit according to the example 1.
Figure 4 is a diagram showing definitions of ellipse parameters to determine the shape of an ellipse according to the example 1.
Figure 5 is a diagram showing a display example of a measurement result from the abdomen circumference measurement unit according to the example 1.
Figure 6 is a flowchart showing an example of the procedure performed with the ellipse fitting unit according to an example 2.

### Description of Embodiments

Hereinbelow, examples of the present invention will be described by using the drawings.

### <Example 1>

Example 1 is an embodiment of an ultrasonic diagnosis device, i.e., ultrasonic diagnosis device configured with an ultrasonic probe part which transmits and receives an ultrasonic wave; an image processing unit which generates a tomographic image of a tissue in a test subject based on a signal obtained from the ultrasonic probe part; an abdomen circumference measurement unit which generates plural ellipses corresponding to an abdomen of the test subject from the tomographic image and measures the abdomen circumference; and an output unit which displays a measurement result from the abdomen circumference measurement unit, and an operation method for the device.

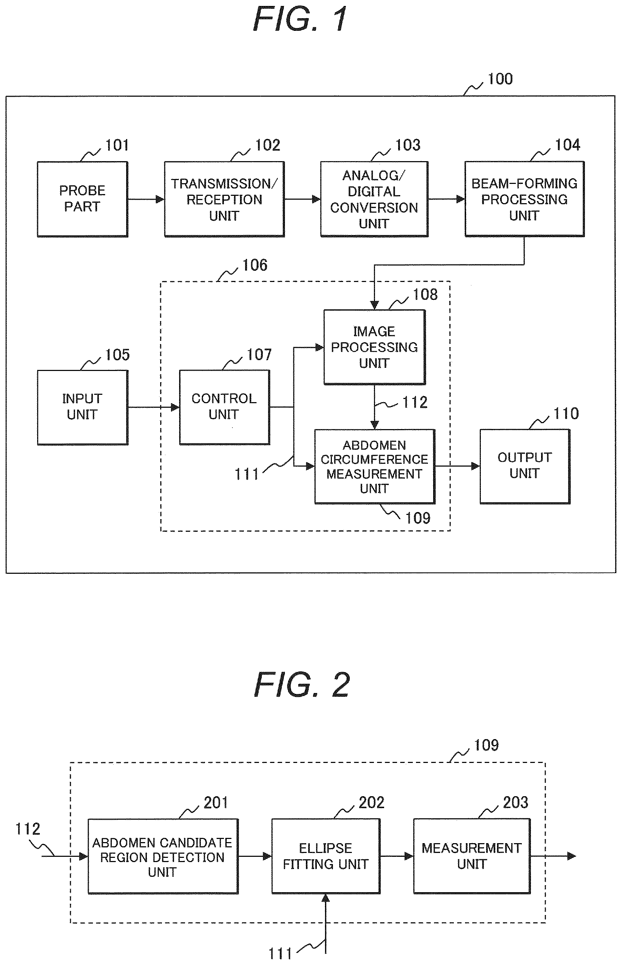

Figure 1 is a block diagram showing an example of a configuration of the ultrasonic diagnosis device according to the present example. An ultrasonic diagnosis device 100 in Figure 1 is configured with a probe part 101 having an ultrasonic oscillator to obtain echo data; a transmission/reception unit 102 to control a transmission pulse and amplify a received echo signal; an analog/digital conversion unit 103 to convert the received signal as an analog signal into a digital signal; a beam-forming processing unit 104 to bundle the received echoes from plural oscillators and perform phase-regulating addition; an image processing unit 108 to perform dynamic range compression, filtering processing and scanning conversion processing with respect to an RF signal from the beam-forming processing unit 104, and generate a sectional image 112 representing the cross-section of the test subject; an abdomen circumference measurement unit 109 to measure the circumference of an abdomen extracted from the sectional image 112; an input unit 105 configured with a touch panel, a keyboard, a track ball and the like, to receive a user's input; a control unit 107 to set parameters 111 in the image processing unit 108 and the abdomen circumference measurement unit 109 based on the user's input; and an output unit 110 to present the measurement result of the circumference of the abdomen or the like, by using a display unit such as a display, to the user. Note that it is possible to realize the control unit 107, the image processing unit 108, and the abdomen circumference measurement unit 109 with a program executed with a central processing unit (CPU) 106 as a processing unit of a general computer. In such case, an input-output unit of the general computer may be utilized as the input unit 105 and the output unit 110.

Hereinbelow, using Figure 2, the abdomen circumference measurement unit 109 will be described. Figure 2 shows an example of the configuration of the abdomen circumference measurement unit 109 in Figure 1. The abdomen circumference measurement unit 109 has three functional blocks, i.e., an abdomen candidate region detection unit 201 to detect a rectangular region including the abdomen from the sectional image 112, an ellipse fitting unit 202 to fit an ellipse to an abdomen contour in the extracted candidate region, and a measurement unit 203 to measure the circumference of the fitted ellipse.

Next, the respective functional blocks of the abdomen circumference measurement unit 109 will be described. The abdomen candidate region detection unit 201 detects a rectangular region circumscribed to the abdomen contour from the sectional image 112 by using recognition processing. For example, as one of recognition processing methods, it uses Adaboost as machine learning. Adaboost is a supervised learning method of connecting a large number of weak discriminators each having low detection accuracy to generate one strong discriminator. Upon learning, a large number of rectangular images including the abdomen and rectangular images not including the abdomen are prepared. Feature amounts extracted from these rectangular images are inputted into an Adaboost learning device. An Adaboost discriminator, generated as a result of learning, discriminates, based on a feature amount of an image given as an input, whether or not the image includes the abdomen. As the feature amount, Haar-like is used. Haar-like is a feature amount to quantify a luminance difference between adjacent pixels.

Note that the discriminator used here may be a discriminator other than Adaboost, e.g., Random Forest, SVM (Support Vector Machine) or the like. Further, the feature amount used here may be other feature amount than Haar-like, e.g., LBP (Local Binary Pattern), HOG (Histogram of Oriented Gradients) or the like. Further, a method using feature amount extraction and a discriminator, integrated with each other, such as CNN (Convolutional Neural Network) as one of Deep Learning methods, may be used.

The abdomen candidate region detection unit 201 inputs an arbitrary rectangular region in the sectional image 112 into the learning device that learned as above, thus has a function of detecting a rectangular region circumscribed to the abdomen contour by determining whether or not the abdomen is included. The ellipse fitting unit 202 has a function of extracting feature points in the rectangular region detected with the abdomen candidate region detection unit 201, and fitting an ellipse to the feature points.

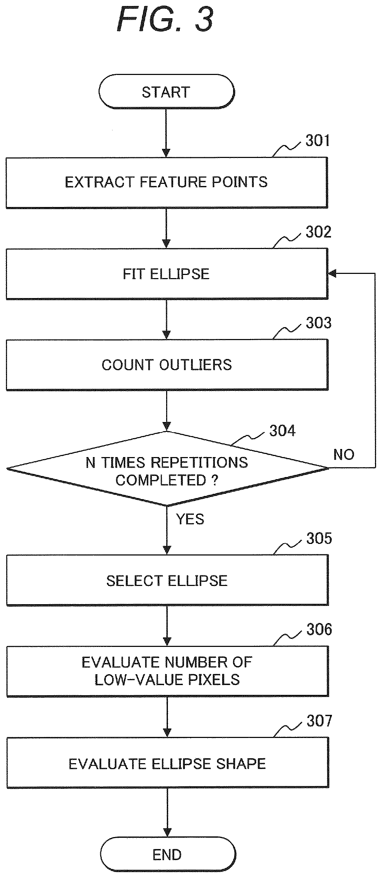

Figure 3 is a flowchart showing an example of a procedure performed with the ellipse fitting unit 202. First, step 301 shows processing of extracting feature points in the rectangular region detected with the abdomen candidate region detection unit 201. As a feature point, a pixel in which an edge extracted with e.g. a Sobel filter exists is used. Since this feature point is regarded as an abdomen contour at step 302 following step 301, it is desirable that it is possible to extract an edge in every direction. That is, as edge extraction means at step 301, other means than the Sobel filter may be used as long as it has an effect of edge extraction in every direction. Note that for the sake of characteristic of the device, it may be configured such that when an edge in a particular direction especially appears strongly in the abdomen contour, to limit the edge extraction direction, the edge extraction direction is changed based on the user's instruction from the input unit 105 or the like. As a feature point, in addition to a pixel where an edge exists, a pixel having a pixel value equal to or higher than a predetermined value may be used.

Step 302 shows processing of selecting arbitrary five or more of feature points extracted at step 301 and fitting an ellipse. As a method of fitting an ellipse, the least-squares method and the Hough transformation are known, but any method may be used. For example, an RANSAC (RANdom SAmple Consensus) algorithm may be used. Regarding the fitted ellipse, for reproduction at step 303 to be described later, ellipse parameters such as the center coordinates, the lengths of short axis and long axis, and the inclination of the long axis are recorded. Note that it may be configured such that when the center coordinates exist outside the sectional image 112 or when a part of the ellipse passes outside the sectional image 112, it is determined that the ellipse is inappropriate as an abdomen contour, and the selection of feature points is performed again. Further, it may be configured such that as a feature point selection method, a clustering method such as k-means is used for selecting feature points more appropriate as an abdomen contour.

Step 303 shows processing of counting the number of outliers with respect to the ellipse fitted at step 302. Note that the outlier means a feature point out of the path of the fitted ellipse. When the number of outliers is smaller, the obtained ellipse matches more feature points. The combination between the ellipse parameters such as the center coordinates, the short-axis and long-axis lengths and the inclination of the long axis, and the number of outliers, is recorded, then, the outlier count processing at step 303 ends.

Step 304 shows processing of determining whether or not a previously-determined number (N) of ellipses have been generated. When the number of ellipses is short, the process returns to step 302 to generate ellipses. When the generation has been completed by the predetermined number (N), the process proceeds to step 305. Note that it is desirable that as the predetermined number, a sufficiently large number, e.g., 1000 times, is set; however, a number variable by the user's input may be set.

Step 305 shows processing of selecting an ellipse with a small number of outliers from the ellipses generated from step 302 to step 304. For example, top three ellipses having a small number of outliers are selected. Note that the top three ellipses are selected here; however, the only top or top four ellipses may be selected. Further, the number of selected ellipses may be a number variable by the user's input.

Step 306 shows processing of comparing the ellipse selected at step 305 with the sectional image 112, counting pixels having a pixel value equal to or lower than a threshold value on the ellipse path, and calculating the percentage with respect to the number of all the pixels on the path. An abdomen contour generally has a pixel value equal to or higher than a fixed value. In the sectional image 112, a region having a small pixel value may be considered as an amniotic fluid region where a fetus abdomen or mother's tissue does not exist. Accordingly, when the number of low value pixels is small on the ellipse path, it may be regarded that the generated ellipse is more suitable as an abdomen contour. For example, when the percentage of the lowest value pixels where the luminosity as a pixel value is 0 or 1 is 20 % or lower with respect to the number of all the pixels on the ellipse path, the ellipse is determined as an appropriate ellipse. Note that it may be configured such that the threshold value of pixel value and the threshold value for determination of the percentage to the number of all the pixels on the ellipse path are variable by the user's input. Further, the determination threshold value may be automatically selected in accordance with the number of pregnancy weeks of the test subject.

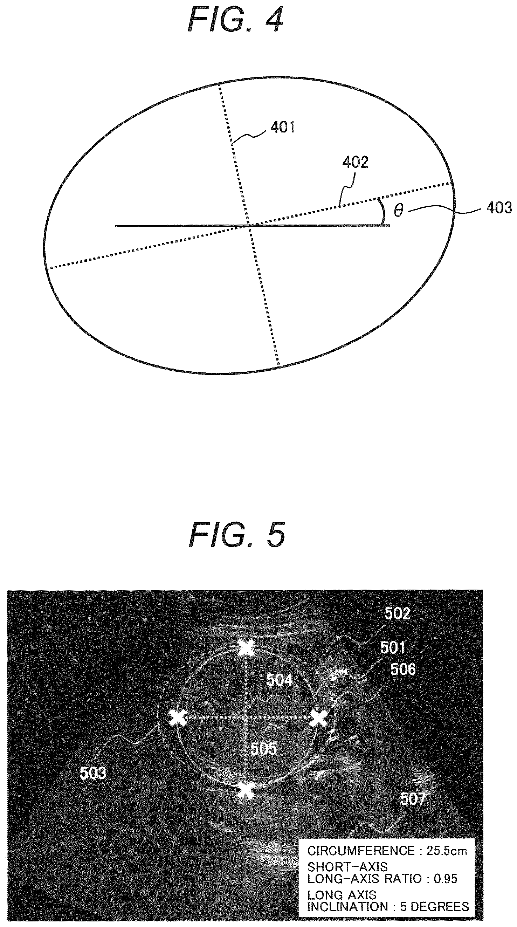

Step 307 shows processing of evaluating shape appropriateness of the ellipse selected at step 305 such as a short-axis long-axis ratio and the inclination of the long axis. Figure 4 is a diagram showing the short axis and the long axis, the inclination of the long axis of the ellipse. The abdomen contour of a fetus primarily has an approximate perfect circular shape. As the pregnancy weeks elapse and the fetus grows, it occupies inside the womb and is pressed with the mother's tissues, the abdomen contour becomes somewhat flat. In consideration of this characteristic, when the ratio of the short axis 401 to the long axis 402 is 0.8 to 1.0, it is determined that the ellipse is an appropriate ellipse as the fetus abdomen contour. Further, in a general checkup, as the mother is in a supine position, the fetus abdomen is pressed from the up and down directions with the mother's tissues. Accordingly, generally, in the sectional image 112, the long axis 401 of the abdomen contour is in an approximately horizontal direction indicated with a solid line in Figure 4. In consideration of this characteristic, when the inclination θ403 of the long axis is equal to or narrower than 45 degrees, the ellipse is determined as an appropriate ellipse. The determination threshold values for the above-described short-axis to long-axis ratio and the inclination of the long axis may be variable by the user's input, or may be automatically selected in accordance with the number of pregnancy weeks.

In Figure 2, the measurement unit 203 of the abdomen circumference measurement unit 109 measures the circumference by using the ellipse parameters such as the center coordinates, the lengths of the short axis and the long axis, and the inclination of the long axis, generated with the ellipse fitting unit 202.

The output unit 110 in Figure 1 performs screen display based on the measurement result from the abdomen circumference measurement unit 109. Figure 5 is a diagram showing an example where three ellipses selected with the abdomen circumference measurement unit 109 as top three ellipses having a small number of outliers, are overlaid on the sectional image 112, and is displayed on the output unit 110. As shown in the figure, by performing display where an ellipse 501 with the smallest number of outliers is emphasized, it is easy to make a comparison with other ellipses 502 and 503, and the convenience for the user is improved. Further, with respect to the ellipse 501 having the smallest number of outliers, by displaying short axis 504, long axis 505, intersections (calipers) 506 between the short axis 504 and the long axis 505 and the ellipse path, the user visually determines the appropriateness of the selected ellipse. Further, by displaying the circumference of the ellipse 501, the short-axis long-axis ratio, and the inclination of the long axis, as a measurement result 507, it is possible to assist quantitative determination.

It may be configured such that when the ellipse is not an appropriate ellipse based on the determination results at step 306 and step 307, a warning message to call the user's attention is displayed. Further, it may be configured such that when it is determined that all the three ellipses selected with the abdomen circumference measurement unit 109 at step 306 and step 307 are not appropriate, it is determined that it is difficult to perform measurement on the sectional image 112 and a warning message to press the user to re-obtain a sectional image is displayed on the output unit 110.

It may be configured such that when the user determines from the display result as described above that the ellipse 501 is not appropriate, the emphasis-displayed ellipse is sequentially selected by the user from the ellipse 501 to the ellipse 502, then to the ellipse 503, by operation of the keyboard or track ball of the input unit 105. Note that it is desirable that when the emphasis-displayed ellipse is changed by the user's operation, the short axis 504, the long axis 505, the calipers 506, and the measurement result 507 are updated in accordance with the change of ellipse. Further, it may be configured such that when the user determines all the ellipses are not appropriate, the user's re-measurement instruction through button operation or the like in the input unit 105 is received, and the processing at step 301 to step 307 is performed again. Upon re-measurement, by performing the ellipse fitting processing at step 302 limitedly to feature points around the ellipse emphasis-displayed by the user before the re-measurement, it is possible to obtain a result closer to the ellipse expected by the user.

With the configuration of the ultrasonic diagnosis device in the above-described example 1, it is possible to display plural ellipse candidates appropriate as an abdomen contour by the fitting processing. Accordingly, when the abdomen contour is not clear, it is possible for the user to obtain a measurement result of the circumference of the abdomen contour with a more simple procedure.

Note that the present example has been explained on a sectional image of a fetus abdomen; however, the invention is applicable to a method for fitting an ellipse to a head contour in a sectional image of a fetus head and measuring the short axis, the long axis and the circumference.

### <Example 2>

An example 2 is an example of the ultrasonic diagnosis device which enables the user to easily correct a measurement result when an expected measurement result has not been outputted in the example 1.

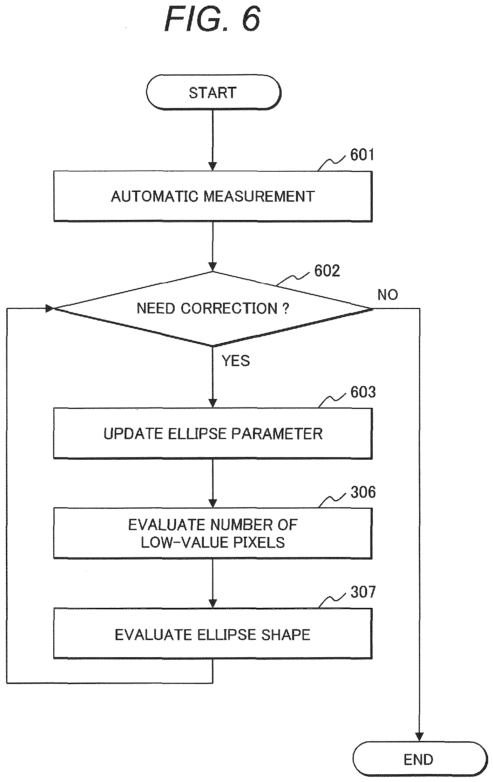

Referring to Figure 6, only the blocks changed from the example 1 will be shown below.

The ellipse fitting unit 202 of the abdomen circumference measurement unit 109 according to the present example automatically measures the abdomen circumference by a similar method to that in the example 1, then performs correction of the measurement result in accordance with a correction request from the user.

Figure 6 shows an example of a flowchart explaining the procedure performed with the ellipse fitting unit 202 in the present embodiment. Step 601 shows processing of performing a series of automatic measurement processing with the ellipse fitting unit 202 in the example 1. That is, the processing shown in the flowchart of Figure 3 is performed from the start to the end.

Step 602 shows processing of confirming whether or not correction of the measurement result is required with respect to the user. At step 602, the output unit 110 inquires the user about execution/non-execution of correction. The user's instruction is received through the input unit 105 and the control unit 107. When an instruction from the user that correction is not necessary (No) is obtained, the present flow ends. The currentlydisplayed measurement result is determined as an abdomen circumference.

Step 603 shows processing of receiving a request for execution of correction (Yes), i.e., an instruction to change the shape of the emphasis-displayed ellipse from the user, at step 602, and updating the ellipse parameters represented as the center coordinates, the lengths of the short axis and the long axis, and the inclination of the long axis of the ellipse. For example, the user gives an instruction to change the shape of the ellipse by moving the calipers 506 by using the track ball or the like of the input unit 105.

At step 306 and step 307, the same processing as that in the example 1 is performed. The measurement unit 203 measures the circumference again with respect to the changed ellipse by performing the same processing as that in the example 1.

With the above-described configuration of the example 2, it is possible for the user to change the positions of the calipers 506 with a simple operation. Further, as the shape of the ellipse is automatically changed and the abdomen circumference is re-calculated, it is possible to easily correct the measurement result.

Further, it may be configured such that the output unit 110 updates the measurement result 507 with respect the ellipse changed by the user. With this configuration, it is possible for the user to move the calipers 506 while checking the measurement result, and to easily correct the measurement result.

Further, it may be configured such that it is possible to change the shape of the ellipse by the user's correcting the numerical values of the measurement result 507, and the result, reflected in the ellipse 501, the short axis 504, the long axis 505 and the calipers 506, is displayed. For example, by changing the short-axis long-axis ratio by 0.01, or changing the inclination of the long axis by 1 degree, it is possible to make a change instruction simpler in comparison with the operation with the calipers 506.

Further, the example where a program realizing a part or all the above-described respective constituents, functions, processors, a controller, an image processing unit and the like, is generated, has been explained, however, they may be realized with hardware by designing a part or all of them with an e.g. integrated circuit. That is, all or a part of the functions of the processors may be replaced with a program, and realized with an integrated circuit such as an ASIC (Application Specific Integrated Circuit) or FPGA (Field Programmable Gate Array) .

### Reference Signs List

- 101:: probe part
- 102:: transmission/reception unit
- 103:: analog/digital conversion unit
- 104:: beam-forming processing unit
- 105:: input unit
- 106:: central processing unit
- 107:: control unit
- 108:: image processing unit
- 109:: abdomen circumference measurement unit
- 110:: output unit
- 201:: abdomen candidate region detection unit
- 202:: ellipse fitting unit
- 203:: measurement unit

## Claims

1. An ultrasonic diagnosis device (100) comprising:
an ultrasonic probe part (101) that transmits/receives an ultrasonic wave;
an image processing unit (108) that generates a tomographic image of a tissue in a test subject based on a signal obtained from the ultrasonic probe part;
an abdomen circumference measurement unit (109) that generates a plurality of ellipses corresponding to an abdomen of the test subject from the tomographic image, and measures a circumference of the abdomen; and
an output unit (110) that displays a measurement result from the abdomen circumference measurement unit,
wherein the abdomen circumference measurement unit (109) comprises
an abdomen candidate region detection unit (201) to detect a rectangular region including the abdomen from the tomographic image,
wherein the rectangular region is detected by using recognition processing using machine learning,
an ellipse fitting unit (202) to fit an ellipse to an abdomen contour in the extracted candidate region, and
a measurement unit (203) to measure the circumference of the fitted ellipse.

2. The ultrasonic diagnosis device according to claim 1,
wherein the abdomen circumference measurement unit generates the plurality of ellipses by repeating processing of extracting feature points which exist in the tomographic image, and fitting an ellipse using arbitrary five of the extracted feature points.

3. The ultrasonic diagnosis device according to claim 2,
wherein regarding each of the generated plurality of ellipses, the abdomen circumference measurement unit counts the feature points out of an ellipse path, as outliers.

4. The ultrasonic diagnosis device according to claim 3,
wherein the output unit emphasis-displays an ellipse with the smallest number of outliers, and displays additional lines indicating a short axis and a long axis of the emphasis-displayed ellipse and calipers at intersections between the additional lines and the ellipse path.

5. The ultrasonic diagnosis device according to claim 4,
wherein the abdomen circumference measurement unit calculates percentage of the number of pixels having a low pixel value with respect to the number of all the pixels on the ellipse path, a ratio between the short axis and the long axis, and inclination of the long axis, and
wherein the output unit displays the circumference, the percentage, the ratio, and the inclination.

6. The ultrasonic diagnosis device according to claim 5,
wherein the abdomen circumference measurement unit compares the circumference, the percentage, the ratio, and the inclination of the long axis, respectively with a reference value calculated from the number of pregnancy weeks of the test subject, and determines whether or not they are within a predetermined range, and
wherein when any one is not within the predetermined range, the output unit displays warning.

7. The ultrasonic diagnosis device according to claim 4, further comprising an input unit that receives an instruction from a user,
wherein the output unit performs emphasis-display in accordance with an instruction to change selection of the plurality of ellipses from the input unit, and changes and displays the ellipse, on which the additional lines and the calipers are displayed.

8. The ultrasonic diagnosis device according to claim 7,
wherein the abdomen circumference measurement unit generates an ellipse based on the positions of the calipers moved based on a moving instruction to move the calipers from the input unit, and calculates the circumference of the ellipse, the percentage of the number of pixels having a low pixel value with respect to the number of all pixels on the ellipse path, the ratio between the short axis and the long axis, and the inclination of the long axis, and
wherein the output unit update-displays the calculated circumference, the percentage, the ratio, and the inclination of the long axis.

9. An operation method for an ultrasonic diagnosis device (100) having a processor, an output unit (110), and an input unit (105),
wherein the processor generates a plurality of ellipses corresponding an abdomen of a test subject, from a tomographic image of a tissue in the test subject, generated based on a signal obtained from an ultrasonic probe part that transmits/receives an ultrasonic wave, measures the circumference of the abdomen a plural number of times, and outputs a plurality of measurement results to the output unit,
the method comprising
detecting, by an abdomen candidate region detection unit (201), a rectangular region including the abdomen from the tomographic image,
wherein detecting the rectangular region is performed by using recognition processing using machine learning,
fitting, by an ellipse fitting unit (202), an ellipse to an abdomen contour in the extracted candidate region, and
measuring, by a measurement unit (203), the circumference of the fitted ellipse.

10. The operation method for the ultrasonic diagnosis device according to claim 9,
wherein the processor generates the plurality of ellipses by repeating processing of extracting feature points which exist in the tomographic image and fitting an ellipse using arbitrary five of the extracted feature points.

11. The operation method for the ultrasonic diagnosis device according to claim 10,
wherein regarding each of the generated plurality of ellipses, the processor counts the feature points out of an ellipse path, as outliers, emphasis-displays an ellipse having the smallest number of outliers on the output unit, and displays additional lines indicating a short axis and a long axis of the emphasis-displayed ellipse and calipers at intersections between the additional lines and the ellipse path.

12. The operation method for the ultrasonic diagnosis device according to claim 11,
wherein the processor calculates the percentage of the number of pixels having a low pixel value with respect to the number of all pixels on the ellipse path, the ratio between the short axis and the long axis, and the inclination of the long axis, and displays the circumference, the percentage, the ratio, and the inclination of the long axis, on the output unit.

13. The operation method for the ultrasonic diagnosis device according to claim 12,
wherein the processor compares the circumference, the percentage, the ratio, and the inclination, respectively with a reference value calculated from the number of pregnancy weeks of the test subject, determines whether or not they are within a predetermined range, and when any one is not within the predetermined range, displays warning on the output unit.

14. The operation method for the ultrasonic diagnosis device according to claim 11,
wherein the processor performs emphasis-display on the output unit in accordance with an instruction to change selection of the plurality of ellipses from the input unit, and changes and displays the ellipse on which the additional lines and the caliper are displayed.

15. The operation method for the ultrasonic diagnosis device according to claim 14,
wherein the processor generates an ellipse based on the position of the calipers based on a moving instruction to move the calipers from the input unit, calculates the circumference of the ellipse, the percentage of the number of pixels having a low pixel value with respect to the number of all pixels on the ellipse path, the ratio between the short axis and the long axis, and the inclination of the long axis, and update-displays the calculated circumference, the percentage, the ratio, and the inclination of the long axis on the output unit.

## Patentansprüche

1. Ultraschalldiagnosevorrichtung (100), die Folgendes umfasst:
ein Ultraschallsondenteil (101), das eine Ultraschallwelle sendet/empfängt;
eine Bildverarbeitungseinheit (108), die ein Tomographiebild eines Gewebes in einem Testindividuum basierend auf einem vom Ultraschallsondenteil erhaltenen Signal erzeugt:
eine Abdomenumfangmesseinheit (109), die eine Vielzahl von Ellipsen, die einem Abdomen des Testindividuums des Tomographiebilds entsprechen, erzeugt und den Umfang des Abdomens misst; und
eine Ausgabeeinheit (110), die ein Messergebnis der Abdomenumfangmesseinheit anzeigt,
wobei die Abdomenumfangmesseinheit (109) Folgendes umfasst:
eine Abdomenkandidatenregiondetektionseinheit (201) zum Detektieren einer rechteckigen Region, die das Abdomen des Tomographiebilds beinhaltet,
wobei die rechteckige Region durch Verwenden von Erkennungsverarbeitung unter Verwendung von Maschinenlernen detektiert wird,
eine Ellipsenanpassungseinheit (202) zum Anpassen einer Ellipse an einen Abdomenumriss in der extrahierten Kandidatenregion,
eine Messeinheit (203) zum Messen des Umfangs der angepassten Ellipse.

2. Ultraschalldiagnosevorrichtung nach Anspruch 1, wobei die Abdomenumfangmesseinheit die Vielzahl von Ellipsen durch Wiederholen des Verarbeitens des Extrahierens von Merkmalpunkten, die im Tomographiebild vorliegen, und des Anpassens einer Ellipse unter Verwendung von beliebigen fünf der extrahierten Merkmalpunkte erzeugt.

3. Ultraschalldiagnosevorrichtung nach Anspruch 2, wobei die Abdomenumfangmesseinheit in Bezug auf jede aus der erzeugten Vielzahl von Ellipsen die Merkmalpunkte aus einem Ellipsenverlauf als Ausreißer zählt.

4. Ultraschalldiagnosevorrichtung nach Anspruch 3, wobei die Ausgabeeinheit die Ellipse mit der geringsten Anzahl an Ausreißern hervorgehoben anzeigt und zusätzliche Linien, die eine kurze Achse und eine lange Achse der hervorgehoben angezeigten Ellipse angeben, sowie Messmarkierungen an Überschneidungen zwischen den zusätzlichen Linien und dem Ellipsenverlauf anzeigt.

5. Ultraschalldiagnosevorrichtung nach Anspruch 4, wobei die Abdomenumfangmesseinheit den Prozentsatz der Anzahl an Pixeln mit niedrigem Pixelwert in Bezug auf die Anzahl aller Pixel auf dem Ellipsenverlauf, das Verhältnis zwischen der kurzen Achse und der langen Achse und die Neigung der langen Achse berechnet und wobei
die Ausgabeeinheit den Umfang, den Prozentsatz, das Verhältnis und die Neigung anzeigt.

6. Ultraschalldiagnosevorrichtung nach Anspruch 5, wobei die Abdomenumfangmesseinheit den Umfang, den Prozentsatz, das Verhältnis und die Neigung der langen Achse jeweils mit einem anhand der Anzahl der Schwangerschaftswochen des Testindividuums berechneten Referenzwert vergleicht und bestimmt, ob sie in einem vorbestimmten Bereich liegen und wobei
die Ausgabeeinheit, wenn eines davon nicht im vordefinierten Bereich liegt, einen Warnhinweis anzeigt.

7. Ultraschalldiagnosevorrichtungseinheit nach Anspruch 4, ferner umfassend eine Eingabeeinheit, die einen Befehl eines Benutzers annimmt,
wobei die Ausgabeeinheit das hervorgehobene Anzeigen gemäß einem Befehl zum Ändern der Auswahl der Vielzahl von Ellipsen von der Eingabeeinheit durchführt und die Ellipse, auf der die zusätzlichen Linien und Messmarkierungen angezeigt sind, ändert und anzeigt.

8. Ultraschalldiagnosevorrichtung nach Anspruch 7, wobei die Abdomenumfangmesseinheit eine Ellipse basierend auf den Positionen der Messmarkierungen, die basierend auf einem Bewegungsbefehl zum Bewegen der Messmarkierungen von der Eingabeeinheit bewegt werden, erzeugt und den Umfang der Ellipse, den Prozentsatz der Anzahl von Pixeln mit niedrigem Pixelwert in Bezug auf die Anzahl aller Pixel auf dem Ellipsenverlauf, das Verhältnis zwischen der kurzen Achse und der langen Achse und die Neigung der langen Achse berechnet und wobei
die Ausgabeeinheit den/das/die berechnete(n) Umfang, Prozentsatz, Verhältnis und Neigung der langen Achse aktualisiert anzeigt.

9. Betriebsverfahren für eine Ultraschalldiagnosevorrichtung (100), die einen Prozessor, eine Ausgabeeinheit (110) und eine Eingabeeinheit (105) umfasst,
wobei der Prozessor eine Vielzahl von Ellipsen, die einem Abdomen eines Testindividuums entsprechen, anhand eines Tomographiebilds eines Gewebes des Testindividuums, das basierend auf einem Signal erzeugt wird, das von einem Ultraschallsondenteil, der eine Ultraschallwelle sendet/empfängt, erhalten wird, erzeugt, den Umfang des Abdomens mehrere Male misst und eine Vielzahl von Messergebnissen für die Ausgabeeinheit ausgibt,
wobei das Verfahren Folgendes umfasst:
Detektieren einer rechteckigen Region, die das Abdomen des Tomographiebilds beinhaltet, durch eine Abdomenkandidatenregiondetektionseinheit (201),
wobei das Detektieren der rechteckigen Region durch Verwenden von Erkennungsverarbeitung unter Verwendung von Maschinenlernen erfolgt,
Anpassen einer Ellipse an einen Abdomenumriss in der extrahierten Kandidatenregion durch eine Ellipsenanpassungseinheit (202) und
Messen des Umfangs der angepassten Ellipse durch eine Messeinheit (203).

10. Betriebsverfahren für eine Ultraschalldiagnosevorrichtung nach Anspruch 9,
wobei der Prozessor die Vielzahl von Ellipsen durch Wiederholen des Verarbeitens des Extrahierens von Merkmalpunkten, die im Tomographiebild vorliegen, und des Anpassens einer Ellipse unter Verwendung von beliebigen fünf der extrahierten Merkmalpunkte erzeugt.

11. Betriebsverfahren für eine Ultraschalldiagnosevorrichtung nach Anspruch 10,
wobei der Prozessor in Bezug auf jede aus der erzeugten Vielzahl von Ellipsen die Merkmalpunkte aus einem Ellipsenverlauf als Ausreißer zählt, eine Ellipse mit der geringsten Anzahl an Ausreißern auf der Ausgabeeinheit hervorgehoben anzeigt und zusätzliche Linien, die eine kurze Achse und eine lange Achse der hervorgehoben angezeigten Ellipse angeben, sowie Messmarkierungen an Überschneidungen zwischen den zusätzlichen Linien und dem Ellipsenverlauf anzeigt.

12. Betriebsverfahren für eine Ultraschalldiagnosevorrichtung nach Anspruch 11,
wobei der Prozessor den Prozentsatz der Anzahl an Pixeln mit niedrigem Pixelwert in Bezug auf die Anzahl aller Pixel auf dem Ellipsenverlauf, das Verhältnis zwischen der kurzen Achse und der langen Achse und die Neigung der langen Achse berechnet und den Umfang, den Prozentsatz, das Verhältnis und die Neigung der langen Achse auf der Ausgabeeinheit anzeigt.

13. Betriebsverfahren für eine Ultraschalldiagnosevorrichtung nach Anspruch 12,
wobei der Prozessor den Umfang, den Prozentsatz, das Verhältnis und die Neigung jeweils mit einem anhand der Anzahl der Schwangerschaftswochen des Testindividuums berechneten Referenzwert vergleicht und bestimmt, ob sie in einem vorbestimmten Bereich liegen und, wenn eines davon nicht im vordefinierten Bereich liegt, auf der Ausgabeeinheit einen Warnhinweis anzeigt.

14. Betriebsverfahren für eine Ultraschalldiagnosevorrichtung nach Anspruch 11,
wobei der Prozessor das hervorgehobene Anzeigen auf der Ausgabeeinheit gemäß einem Befehl zum Ändern der Auswahl der Vielzahl von Ellipsen von der Eingabeeinheit durchführt und die Ellipse, auf der die zusätzlichen Linien und Messmarkierungen angezeigt sind, ändert und anzeigt.

15. Betriebsverfahren für eine Ultraschalldiagnosevorrichtung nach Anspruch 14,
wobei der Prozessor eine Ellipse basierend auf den Positionen der Messmarkierungen, die basierend auf einem Bewegungsbefehl zum Bewegen der Messmarkierungen von der Eingabeeinheit bewegt werden, erzeugt und den Umfang der Ellipse, den Prozentsatz der Anzahl von Pixeln mit niedrigem Pixelwert in Bezug auf die Anzahl aller Pixel auf dem Ellipsenverlauf, das Verhältnis zwischen der kurzen Achse und der langen Achse und die Neigung der langen Achse berechnet und den/das/die berechnete(n) Umfang, Prozentsatz, Verhältnis und Neigung der langen Achse auf der Ausgabeeinheit aktualisiert anzeigt.

## Revendications

1. Dispositif de diagnostic par ultrasons (100) comprenant :
une partie de sonde ultrasonore (101) qui émet/reçoit une onde ultrasonore ;
une unité de traitement d'image (108) qui génère une image tomographique d'un tissu dans un sujet de test sur la base d'un signal obtenu à partir de la partie de sonde ultrasonore ;
une unité de mesure de circonférence d'abdomen (109) qui génère une pluralité d'ellipses, correspondant à un abdomen du sujet de test, à partir de l'image tomographique, et mesure une circonférence de l'abdomen ; et
une unité de sortie (110) qui affiche un résultat de mesure provenant de l'unité de mesure de circonférence d'abdomen ;
dans laquelle l'unité de mesure de circonférence d'abdomen (109) comprend :
une unité de détection de région candidate d'abdomen (201) destinée à détecter une région rectangulaire incluant l'abdomen, à partir de l'image tomographique,
dans laquelle la région rectangulaire est détectée en utilisant un traitement de reconnaissance faisant appel à l'apprentissage automatique ;
une unité d'ajustement d'ellipse (202) destinée à ajuster une ellipse à un contour d'abdomen dans la région candidate extraite ; et
une unité de mesure (203) destinée à mesurer la circonférence de l'ellipse ajustée.

2. Dispositif de diagnostic par ultrasons selon la revendication 1,
dans lequel l'unité de mesure de circonférence d'abdomen génère la pluralité d'ellipses en répétant un traitement consistant à extraire des points caractéristiques qui existent dans l'image tomographique, et à ajuster une ellipse en utilisant cinq points caractéristiques arbitraires des points caractéristiques extraits.

3. Dispositif de diagnostic par ultrasons selon la revendication 2,
dans lequel, en ce qui concerne chacune de la pluralité d'ellipses générée, l'unité de mesure de circonférence d'abdomen compte les points caractéristiques, situés en dehors d'une trajectoire d'ellipse, comme étant des valeurs aberrantes.

4. Dispositif de diagnostic par ultrasons selon la revendication 3,
dans lequel l'unité de sortie affiche en accentuation une ellipse présentant le plus petit nombre de valeurs aberrantes, et affiche des lignes supplémentaires indiquant un axe court et un axe long de l'ellipse affichée en accentuation, et des calibres à coulisse aux intersections entre les lignes supplémentaires et la trajectoire d'ellipse.

5. Dispositif de diagnostic par ultrasons selon la revendication 4,
dans lequel l'unité de mesure de circonférence d'abdomen calcule un pourcentage du nombre de pixels présentant une valeur de pixel faible par rapport au nombre de tous les pixels sur la trajectoire d'ellipse, un rapport entre l'axe court et l'axe long, et l'inclinaison de l'axe long ; et
dans lequel l'unité de sortie affiche la circonférence, le pourcentage, le rapport et l'inclinaison.

6. Dispositif de diagnostic par ultrasons selon la revendication 5,
dans lequel l'unité de mesure de circonférence d'abdomen compare la circonférence, le pourcentage, le rapport et l'inclinaison de l'axe long, respectivement, à une valeur de référence calculée à partir du nombre de semaines de grossesse du sujet de test, et détermine si ces éléments se situent ou non dans une plage prédéterminée ; et
dans lequel, lorsque l'un quelconque desdits éléments ne se situe pas dans la plage prédéterminée, l'unité de sortie affiche un avertissement.

7. Dispositif de diagnostic par ultrasons selon la revendication 4, comprenant en outre une unité d'entrée qui reçoit une instruction en provenance d'un utilisateur,
dans lequel l'unité de sortie met en œuvre un affichage en accentuation conformément à une instruction de modification d'une sélection de la pluralité d'ellipses provenant de l'unité d'entrée, et modifie et affiche l'ellipse sur laquelle les lignes supplémentaires et les calibres à coulisse sont affichés.

8. Dispositif de diagnostic par ultrasons selon la revendication 7,
dans lequel l'unité de mesure de circonférence d'abdomen génère une ellipse sur la base des positions des calibres à coulisse déplacés sur la base d'une instruction de déplacement des calibres à coulisse provenant de l'unité d'entrée, et calcule la circonférence de l'ellipse, le pourcentage du nombre de pixels présentant une valeur de pixel faible par rapport au nombre de tous les pixels sur la trajectoire d'ellipse, le rapport entre l'axe court et l'axe long, et l'inclinaison de l'axe long ; et
dans lequel l'unité de sortie affiche de manière actualisée la circonférence calculée, le pourcentage calculé, le rapport calculé et l'inclinaison calculée de l'axe long.

9. Procédé de fonctionnement pour un dispositif de diagnostic par ultrasons (100) présentant un processeur, une unité de sortie (110), et une unité d'entrée (105),
dans lequel le processeur génère une pluralité d'ellipses correspondant à un abdomen d'un sujet de test, à partir d'une image tomographique d'un tissu dans le sujet de test, générée sur la base d'un signal obtenu à partir d'une partie de sonde ultrasonore qui émet/reçoit une onde ultrasonore, mesure la circonférence de l'abdomen une pluralité de fois, et fournit en sortie une pluralité de résultats de mesure à l'unité de sortie ;
le procédé comprenant les étapes ci-dessous consistant à :
détecter, par le biais d'une unité de détection de région candidate d'abdomen (201), une région rectangulaire incluant l'abdomen, à partir de l'image tomographique ;
dans lequel l'étape de détection de la région rectangulaire est mise en œuvre en utilisant un traitement de reconnaissance faisant appel à l'apprentissage automatique ;
ajuster, par le biais d'une unité d'ajustement d'ellipse (202), une ellipse, à un contour d'abdomen, dans la région candidate extraite ; et
mesurer, par le biais d'une unité de mesure (203), la circonférence de l'ellipse ajustée.

10. Procédé de fonctionnement pour le dispositif de diagnostic par ultrasons selon la revendication 9,
dans lequel le processeur génère la pluralité d'ellipses en répétant un traitement consistant à extraire des points caractéristiques qui existent dans l'image tomographique et à ajuster une ellipse en utilisant cinq points caractéristiques arbitraires des points caractéristiques extraits.

11. Procédé de fonctionnement pour le dispositif de diagnostic par ultrasons selon la revendication 10,
dans lequel, en ce qui concerne chacune de la pluralité d'ellipses générée, le processeur compte les points caractéristiques, situés en dehors d'une trajectoire d'ellipse, comme étant des valeurs aberrantes, affiche en accentuation une ellipse présentant le plus petit nombre de valeurs aberrantes sur l'unité de sortie, et affiche des lignes supplémentaires indiquant un axe court et un axe long de l'ellipse affichée en accentuation, et des calibres à coulisse aux intersections entre les lignes supplémentaires et la trajectoire d'ellipse.

12. Procédé de fonctionnement pour le dispositif de diagnostic par ultrasons selon la revendication 11,
dans lequel le processeur calcule le pourcentage du nombre de pixels présentant une valeur de pixel faible par rapport au nombre de tous les pixels sur la trajectoire d'ellipse, le rapport entre l'axe court et l'axe long, et l'inclinaison de l'axe long, et affiche la circonférence, le pourcentage, le rapport et l'inclinaison de l'axe long, sur l'unité de sortie.

13. Procédé de fonctionnement pour le dispositif de diagnostic par ultrasons selon la revendication 12,
dans lequel le processeur compare la circonférence, le pourcentage, le rapport et l'inclinaison, respectivement à une valeur de référence calculée à partir du nombre de semaines de grossesse du sujet de test, détermine si ces éléments se situent ou non dans une plage prédéterminée, et lorsque l'un quelconque desdits éléments ne se situe pas dans la plage prédéterminée, affiche un avertissement sur l'unité de sortie.

14. Procédé de fonctionnement pour le dispositif de diagnostic par ultrasons selon la revendication 11,
dans lequel le processeur met en œuvre un affichage en accentuation sur l'unité de sortie conformément à une instruction de modification d'une sélection de la pluralité d'ellipses provenant de l'unité d'entrée, et modifie et affiche l'ellipse sur laquelle les lignes supplémentaires et le calibre à coulisse sont affichés.

15. Procédé de fonctionnement pour le dispositif de diagnostic par ultrasons selon la revendication 14,
dans lequel le processeur génère une ellipse sur la base de la position des calibres à coulisse déplacés sur la base d'une instruction de déplacement, visant à déplacer les calibres à coulisse, provenant de l'unité d'entrée, calcule la circonférence de l'ellipse, le pourcentage du nombre de pixels présentant une valeur de pixel faible par rapport au nombre de tous les pixels sur la trajectoire d'ellipse, le rapport entre l'axe court et l'axe long, et l'inclinaison de l'axe long, et affiche de manière actualisée la circonférence calculée, le pourcentage calculé, le rapport calculé et l'inclinaison calculée de l'axe long sur l'unité de sortie.
